# FASCICULE DE BREVET EUROPEEN

(11) **EP 1 141 337 B1**
(45) Date de publication et mention de la délivrance du brevet: **24.11.2010**
(21) Numéro de dépôt: 99961158.5
(22) Date de dépôt: 23.12.1999
(51) Int. Cl.: C12N 15/57, C12N 9/52

(54) **BACTERIES A GRAM POSITIF DEPOURVUES D'ACTIVITE PROTEASIQUE HtrA, ET LEURS UTILISATIONS**
HTRA-PROTEASEAKTIVITÄTFREIEN GRAM-POSITIVEN BAKTERIEN UND IHRE VERWENDUNGEN
GRAM-POSITIVE BACTERIA DEPRIVED OF HtrA PROTEASIC ACTIVITY AND THEIR USES

(30) Priorité: 24.12.1998 FR 9816462
(43) Date de publication de la demande: 10.10.2001
(73) Titulaire: INSTITUT NATIONAL DE LA RECHERCHE AGRONOMIQUE (INRA), 75338 Paris Cédex 07 (FR)
(72) Inventeur: POQUET, Isabelle, F-75015 Paris (FR); GRUSS, Alexandra, F-91400 ORSAY (FR); BOLOTINE, Alexandre, F-54000 Nancy (FR); SOROKINE, Alexei, F-91190 Gif-sur-Yvette (FR)
(74) Mandataire: Orès, Bernard
(86) Numéro de dépôt international: PCT/FR1999/003270
(87) Numéro de publication internationale: WO 2000/039309

(56) Documents cités:
- WO-A-88/05821
- WO-A-91/15572
- US-A- 5 264 365
- ANDRÈAS SMEDS ET AL.: "Molecular characterization of a stress-inducible gene from Lactobacillus helveticus" JOURNAL OF BACTERIOLOGY, vol. 180, no. 23, décembre 1998 (1998-12), pages 6148-6153, XP002113159 cité dans la demande
- PALLEN M J ET AL: "The HtrA family of serine proteases." MOLECULAR MICROBIOLOGY, (1997 OCT) 26 (2) 209-21. REF: 72, XP002113160

## Description

L'invention concerne la production, chez des bactéries à Gram-positif, de protéines exportées.

On désigne sous le terme général de : « protéines exportées », des protéines qui sont transportées à travers la membrane cytoplasmique. Dans le cas des bactéries à Gram-positif, ce transport aboutit à la sécrétion de la protéine dans le milieu, ou à son association à la surface cellulaire.

L'un des principaux problèmes qui se pose lors de la production de protéines d'intérêt exportées par des bactéries-hôtes, réside dans la dégradation de ces protéines pendant et/ou après leur exportation, au niveau de l'enveloppe ou de la surface de la cellule. Cette dégradation entraîne souvent une baisse du rendement, et/ou une altération de la structure et de l'activité de la protéine.

Les enzymes responsables de cette dégradation des protéines exportées, sont des protéases bactériennes elles-mêmes exportées dans l'enveloppe ; il s'agit de protéases dites : « de ménage », qui ont normalement parmi leurs fonctions principales un rôle de dégradation de protéines exportées anormales ou mal repliées s'accumulant dans le milieu ou dans l'enveloppe, notamment en conditions de stress, ainsi qu'un rôle de recyclage des protéines exportées.

Les protéines hétérologues, qui sont souvent imparfaitement reconnues par les protéines chaperons intervenant dans le repliement des protéines chez la bactérie-hôte sont particulièrement sensibles à l'attaque de ces protéases.

La protéase de ménage exportée la plus anciennement caractérisée est la protéase à sérine HtrA/DegP d'*E. coli.* Il s'agit d'une protéase à localisation périplasmique, qui est exprimée sous contrôle d'un promoteur inductible à haute température ; BECKWITH et STRAUCH (Proc. Natl. Acad. Sci. USA 85:1576-1580, 1988) ont observé qu'elle intervenait dans la protéolyse de protéines de fusion entre des protéines exportées d'*E. coli* et le rapporteur de l'exportation PhoA. Ils ont proposé d'inactiver cette protéase chez *E. coli* afin de limiter la dégradation des protéines hétérologues exportées.

Des souches mutantes d'*E. coli,* dans lesquelles le gène codant pour la protéase HtrA/DegP a été inactivé ont ainsi été obtenues [BECKWITH et STRAUCH, publication précitée, et Demande PCT WO88/05821] ; cependant il a été constaté que cette inactivation se traduit par un ralentissement de la cinétique de dégradation, mais ne suffit pas pour l'abolir, du fait de l'existence dans l'enveloppe d'autres protéases dégradant les protéines exportées.

Chez *E. coli,* plusieurs protéases de ménage de l'enveloppe, assurant des fonctions similaires à celles de HtrA/DegP ont été caractérisées : il s'agit notamment des protéases HhoA/DegQ et HhoB/DegS, structurellement homologues à HtrA/DegP, et de protéases de structure différente mais fonctionnellement comparables (ApeA/protéaseI, OmpT, OmpP, Prc/Tsp, SppA/protéaseIV, PrtIII et SohB).

Des études concernant d'autres bactéries ont également permis de mettre en évidence l'existence dans chaque espèce étudiée, de plusieurs protéases de ménage exportées. Par exemple, de très nombreuses espèces bactériennes possèdent plusieurs protéases de la famille HtrA (PALLEN et WREN, Mol. Microbiol. 19:209-21, 1997) ; trois homologues de HtrA ont été identifiés chez *B. subtilis* (YyxA, YkdA et YvtB/Yirf), *Synechocystis* (HtrA, HhoA et HhoB), Pseudomonas *aueruginosa* et *Aquifex aeolicus,* deux chez *Haemophilus influenzae* (HtoA et HhoB), *Campylobacter jejuni, Brucella abortus* et *Yersinia enterolitica,* et quatre chez *Mycobacterium tuberculosis.* Diverses bactéries à Gram-positif possèdent également des protéases à sérine considérées comme apparentées à la famille HtrA, sur la base d'une homologie au niveau du domaine catalytique : EtA, EtB, V8/StsP de *S. aureus,* GseP de *Bacillus licheniformis* et Spro de *Mycobacterium paratuberculosis* (KOONIN et al., Chap 117 in Escherichia coli and Salmonella typhimurium, 2203-17, 1997). Enfin, des protéases exportées non-apparentées à HtrA, ont également été mises en évidence, par exemple chez *B. subtilis* (MARGOT et KARAMATA, Microbiology, 142:3437-44, 1996 ; STEPHENSON et HARWOOD Appl. Environn. Microbiol. 64:2875-2881, 1998 ; WU et al. J. Bacteriol. 173:4952-58, 1991).

Il a donc été proposé de combiner des mutations affectant plusieurs protéases exportées, pour parvenir à une réduction effective de la dégradation des protéines hétérologues exportées.

Par exemple, une souche d'*E. coli* mutée dans les gènes *degP*/*htrA, ompT,* prt et *prc* (MEERMAN et GEORGIOU, Bio/technology 12:1107-10, 1994), et une souche de *B. subtilis* déficiente dans les six protéases extracellulaires (WU *et al.,* 1991, publication précitée), ont été construites dans ce but. Cependant, l'utilisation de ces souches ne permet pas d'éliminer totalement la protéolyse des protéines exportées. Par exemple, dans le cas de la souche de *B. subtilis* décrite par WU *et al.,* bien que l'activité protéasique extracellulaire résiduelle soit négligeable (<1%), la dégradation des protéines hétérologues exportées reste importante. Pour pallier ce problème, cette même équipe a apporté des modifications supplémentaires à cette souche, afin de lui faire surproduire divers chaperons (WU et al., J. Bacteriol. 180:2830-35, 1998). En outre, bien que l'inactivation du gène d'une de ces protéases de ménage exportées n'ait pas de conséquences notables sur la bactérie, le cumul des mutations peut affecter la viabilité des souches ; MEERMAN et GEORGIOU, (1994, publication précitée) observent ainsi une diminution du taux de croissance pouvant aller jusqu'à 50%.

Chez les bactéries lactiques, seules quelques protéases exportées ont fait l'objet d'études ; la mieux caractérisée à l'heure actuelle est la protéase dénommée PrtP (KOK, FEMS Microbiol. Reviews 87:15-42, 1990), qui est localisée à la surface cellulaire, où elle est ancrée au peptidoglycanne. Cette protéase est présente chez de nombreuses bactéries lactiques, notamment *Lactococcus lactis,* où son gène est plasmidique. Elle participe à la nutrition azotée des bactéries en dégradant les caséines du lait. D'autres protéases de surface ont été purifiées à partir de deux espèces de bactéries lactiques, *Lactobacillus delbrueckeii* subsp. *bulgaricus* et *Lactobacillus helveticus,* mais leur fonction n'a pas été déterminée. (STEFANITSI et al., FEMS Microbiol. Lett. 128:53-8, 1995 ; STEFANITSI et GAREL, Lett. Appl. Microbiol. 24:180-84, 1997 ; YAMAMOTO, et al., J. Biochem. 114:740-45, 1993). Récemment, un gène inductible par le stress codant pour une protéine fortement homologue aux protéases de la famille HtrA a été mis en évidence chez *Lactobacillus* helveticus (SMEDS et al., J. Bacteriol. 180:6148-53, 1998). Il a été observé que ce gène était nécessaire à la survie à température élevée ; une souche mutante de *Lactobacillus helveticus* dans laquelle le gène *htrA* a été inactivé par l'insertion d'un gène rapporteur (*gusA,* codant pour la β-glucuronidase) sous contrôle du promoteur *htrA,* a été construite. L'étude de l'expression du gène *gusA* dans ce mutant a permis de mettre en évidence une induction de la transcription de ce gène dans les mêmes conditions que celle du gène *htrA* dans les souches sauvages ; en revanche, aucune activité β-glucuronidase n'a été observée.

Lors de travaux précédents visant à caractériser des protéines exportées de *Lactococcus lactis* par l'étude de protéines de fusion avec le rapporteur d'exportation Δ_{SP}Nuc (POQUET et al., J. Bacteriol. 180:1904-12, 1998), l'équipe des Inventeurs a observé une importante protéolyse extra-cellulaire, bien que les expérimentations aient été effectuées dans une souche de *L. lactis* subsp. *cremoris* dépourvue de tout plasmide, et donc en particulier de celui qui porte *prtP.*

Les Inventeurs ont entrepris de rechercher des protéases extra-cellulaires responsables de cette protéolyse.

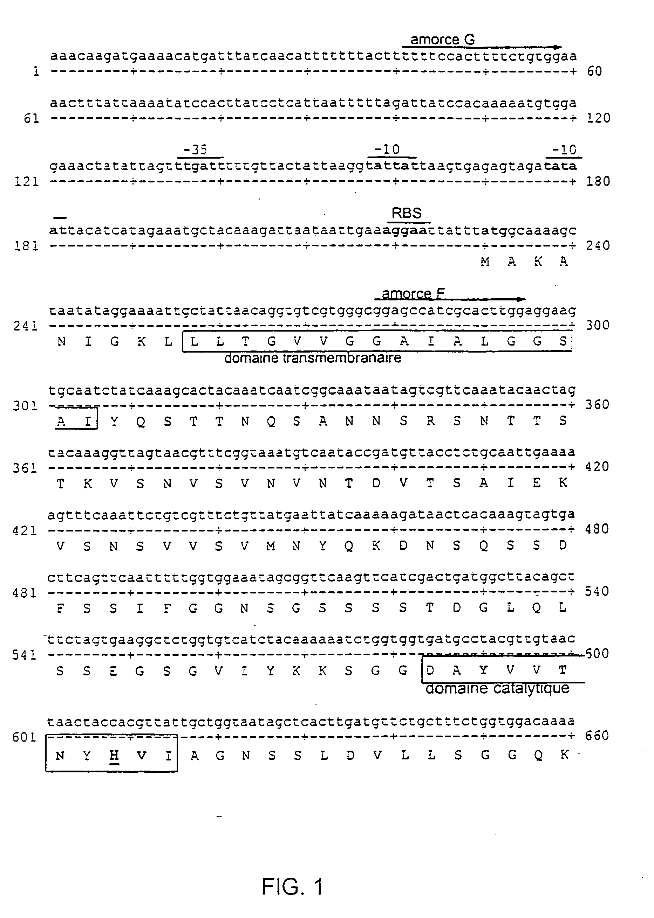

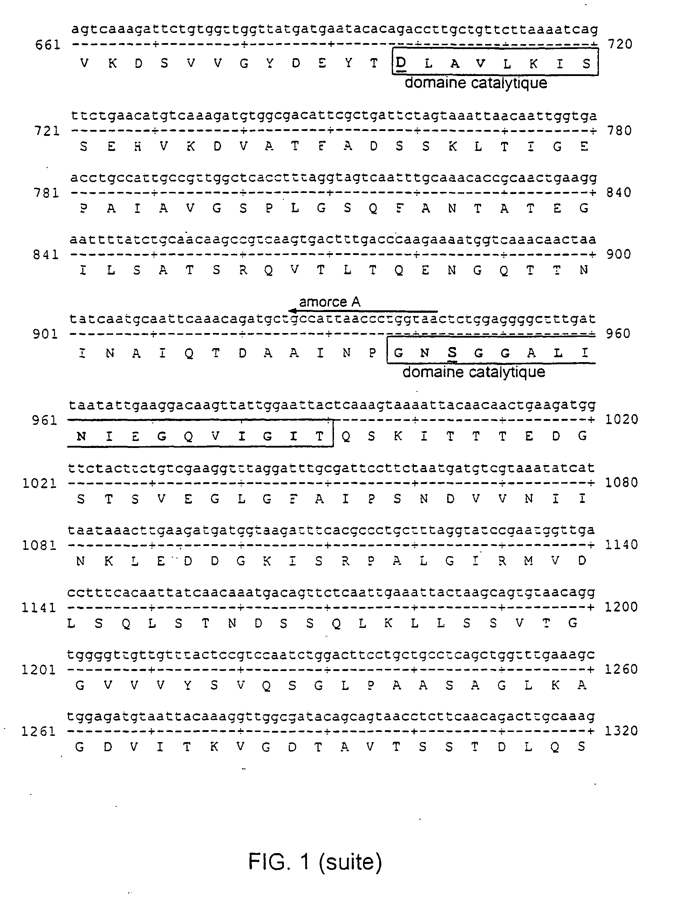

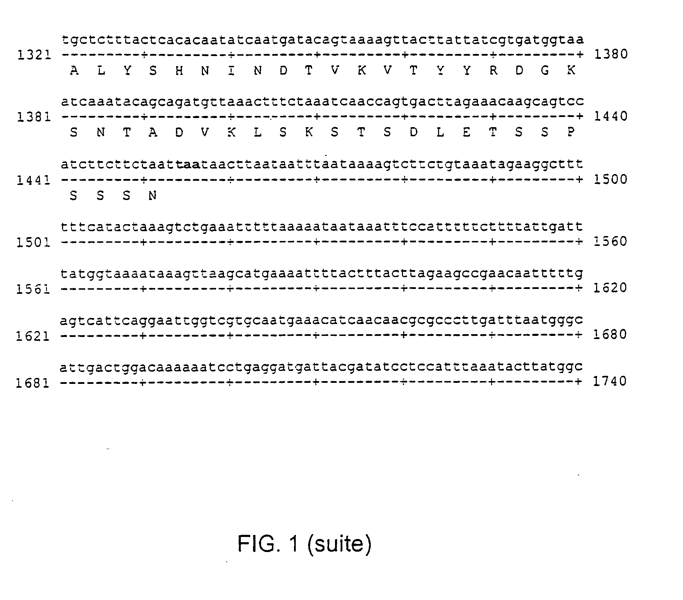

Ils ont ainsi découvert, chez *L. lactis,* l'existence d'un gène de la famille *htrA.* Ce gène, mis en évidence dans le génome de la souche IL1403 de *L. lactis* subsp. *lactis,* code pour une protéine de 408 acides aminés, dénommée ci-après HtrA_{Ll} dont la séquence nucléotidique et la séquence en acides aminés sont représentées sur la Figure 1, et figurent dans la liste de séquences en annexe (SEQ ID NO: 1). Cette protéine est très homologue à HtrA d'*E. coli,* et à divers autres membres connus de la famille HtrA, comme le montre le Tableau I ci dessous, qui illustre les pourcentages d'identité et de similarité entre HtrA_{Ll} et différentes protéines de la famille HtrA :

**TABLEAU I**

| Protéine | Organisme | % identité | % similarité |
|---|---|---|---|
| HtrA/DegP/ protéase Do | *E. coli* | 31.5 | 38.2 |
| HhoA/DegQ | *E. coli* | 34.0 | 40.8 |
| HhoB/DegS | *E. coli* | 29.9 | 37.3 |
| HtrA | *S. typhimurium* | 32.4 | 39.1 |
| HtoA | *H. influenzae* | 31.9 | 39.2 |
| HhoB/DegS | *H. influenzae* | 31.2 | 40.0 |
| spHtrA | *S. pneumoniae* | 55.6 | 62.0 |
| HtrA | *Lb. helveticus* | 46.9 | 54.1 |
| YyxA | *B. subtilis* | 43.5 | 52.0 |
| YkdA | *B. subtilis* | 42.5 | 49.4 |

La protéine HtrA de la souche IL1403 de *L. lactis* subsp. *lactis* possède les trois acides aminés Ser, His et Asp, qui définissent le site catalytique caractéristique des protéases à sérine apparentées à la trypsine, parmi lesquelles la famille HtrA ; en outre elle présente, autour de ces trois acides aminés, les trois motifs suivants : DAYVVTNYH₁₂₇VI, D₁₅₇LAVLKIS, et GNS₂₃₉GGALINIEGQVIGIT, qui correspondent aux consensus définis par PALLEN et WREN (Mol. Microbiol. 19:209-21, 1997) pour le domaine catalytique des protéases HtrA : - GY--TN-HV-, D-AV---- et GNSGG-L-N-G--IGIN.

Elle possède à son extrémité N-terminale une séquence d'acides aminés hydrophobes L₁₀LTGVVGGAIALGGSAI₂₆ correspondant à un segment transmembranaire putatif. La protéine HtrA_{Ll} de *L. lactis* subsp. *lactis* serait donc une protéine intégrale de la membrane cytoplasmique. Selon la règle dite « du positif interne » concernant la topologie de ces protéines (VON HEIJNE, Nature, 341:456-8, 1989) sa topologie correspond au type « C-out », c'est-à-dire que sa partie C-terminale, qui comprend en particulier son site catalytique, serait exposée à l'extérieur de la membrane plasmique. Comme la protéase HtrA de *E. coli,* HtrA_{Ll} de *L. lactis* subsp. *lactis* apparaît donc comme une protéase de l'enveloppe, pouvant dégrader des protéines exportées. Les acides aminés du domaine catalytique et du domaine transmembranaire sont encadrés sur la figure 1.

Les Inventeurs ont procédé à l'inactivation de ce gène par mutation ; à température optimale (30°C), la souche mutante de *L. lactis* subsp. *lactis* ainsi obtenue est viable et croit normalement ; en revanche, sa croissance et sa viabilité sont affectées à plus hautes températures (à partir de 37°C), à la fois sur boite et et en milieu liquide.

En outre, les Inventeurs ont étudié l'effet de cette mutation sur l'exportation de différentes protéines de fusion, et ont constaté que l'inactivation de la protéase HtrA_{Ll} chez *L. lactis* suffisait à abolir totalement la dégradation des protéines exportées ; cet effet est surprenant, compte tenu de la protéolyse résiduelle observée antérieurement chez d'autres bactéries après inactivation de protéases de la famille HtrA.

La présente invention a pour objet un procédé pour la production d'une protéine d'intérêt, **caractérisé en ce qu**'il comprend la mise en culture d'une bactérie exprimant ladite protéine d'intérêt, et l'obtention de ladite protéine d'intérêt exportée par ladite bactérie, **caractérisé en ce que** ladite bactérie est une bactérie à gram positif choisie parmi les *Streptococcaceae,* les *Lactobacillaceae,* les *Bacillaceae,* du genre Listeria, et les *Enterococcaceae,* du genre *Enterococcus,* dont la protéase de surface HtrA est inactivée par mutation. Avantageusement, ladite bactérie est choisie parmi les lactocoques.

Avantageusement, ladite souche bactérienne peut également comporter une ou plusieurs autres modifications de son génome, visant à améliorer la production et/ou la sécrétion de protéines exprimées dans ladite bactérie, et/ou à éviter leur dégradation. Selon le type de protéine que l'on souhaite obtenir, on peut par exemple utiliser une souche de lactocoque dans laquelle l'activité protéasique PrtP a été inactivée, et/ou une souche bactérienne surproduisant une protéine permettant de stabiliser les protéines exportées, telle que la protéine Nlp4 de *Lactococcus lactis,* ou un de ses homologues (POQUET et al. 1998, publication précitée).

La présente invention a également pour objet toute souche bactérienne, susceptible d'être obtenue à partir d'une bactérie à gram positif telle que définie ci-dessus, par mutation inactivant la protéase de surface HtrA de ladite bactérie, et comprenant en outre au moins une cassette d'expression d'un gène d'intérêt, à l'exception d'une souche de *Lactobacillus helveticus* comprenant une seule cassette d'expression, constituée par la séquence codant pour le gène rapporteur *gusA* insérée dans le gène *htrA* de ladite souche, sous contrôle transcriptionnel du promoteur dudit gène.

On entend par : « cassette d'expression » toute construction d'ADN recombinant comprenant un gène d'intérêt que l'on souhaite exprimer, ou un site permettant l'insertion dudit gène, placé sous contrôle de séquences de régulation de la transcription (promoteur, terminateur) fonctionnelles dans la bactérie-hôte concernée.

Au sens de la présente invention, on entend par : « protéase HtrA » toute protéase à sérine de type trypsine, présentant des similitudes fonctionnelles et structurelles suffisantes avec la protéase HtrA de *E. coli,* pour pouvoir être regroupée dans la même famille, à savoir :
- un site catalytique formé par les trois acides aminés Ser, His et Asp ;
- la présence, autour de ce site catalytique, des régions consensus : -GY--TN-HV-, D-AV---- et GNSGG-L-N-G-IGIN ;
- un signal d'exportation permettant à la protéase d'être transportée jusqu'à la surface cellulaire de la bactérie (il peut s'agir, par exemple, d'un peptide signal, d'un domaine transmembranaire, d'un signal d'ancrage à la paroi, etc.).

Pour la mise en oeuvre de la présente invention, on peut obtenir des bactéries mutantes dépourvues d'activité HtrA en effectuant une ou plusieurs mutations, notamment au niveau de la séquence codant pour la protéase HtrA et/ou au niveau des séquences de régulation permettant l'expression du gène *htrA,* de manière à empêcher l'expression d'une protéase HtrA fonctionnelle. Ces mutations peuvent être effectuées de manière classique, par délétion, insertion, ou remplacement d'au moins un nucléotide ou une séquence nucléotidique dans le gène HtrA ; elles peuvent résulter soit en l'absence de production de HtrA, soit en la production d'une protéase HtrA dans laquelle au moins un acide aminé nécessaire à l'activité a été délété ou remplacé.

Les techniques de mutagénèse appropriées sont connues en elles-mêmes ; avantageusement, on utilisera des techniques de mutagénèse dirigée, dans la mesure où les données disponibles sur les protéases de la famille HtrA permettent, même si l'on ne dispose pas d'informations plus précises sur la séquence spécifique du gène que l'on souhaite inactiver, de cibler la ou les mutations sur des domaines conservés nécessaires à l'activité (par exemple le domaine catalytique).

La présente invention peut être mise en oeuvre dans de nombreux domaines.

En premier lieu, elle peut être utilisée dans le domaine de la production de protéines d'intérêt (par exemple enzymes, protéines humaines, etc.) par génie génétique à partir de cultures de bactéries transformées par un gène d'intérêt. Dans ce domaine, la présente invention permet d'améliorer le rendement en protéines exportées (et en particulier sécrétées), et d'éviter leur contamination par des produits de protéolyse, inactifs : ceci permet de les purifier facilement et à moindre coût.

Pour cette application on utilisera de préférence des souches mutantes obtenues à partir de bactéries non-pathogènes, telles que *Lactococcus* spp., *Lactobacillus* spp., ainsi que des streptocoques alimentaires, *Streptococcus thermophilus.*

Les souches mutantes obtenues à partir de bactéries habituellement utilisées en industrie agro-alimentaire, telles que les bactéries lactiques (notamment, les lactocoques, les lactobacilles et les streptocoques thermophiles), peuvent avantageusement être utilisées dans ce même domaine. Par exemple, on peut les utiliser dans la composition de ferments, pour produire des protéines hétérologues permettant d'améliorer la qualité du produit fermenté fini ; ainsi, l'exportation d'enzymes étrangères produites par une souche mutante de *L. lactis* conforme à l'invention, au sein de fromages fermentés par *L. lactis* peut améliorer leur affinage et leurs qualités organoleptiques.

Ces souches mutantes peuvent également être utilisées pour l'obtention de produits diététiques ou de médicaments. Dans ce domaine on peut par exemple utiliser des souches mutantes conformes à l'invention afin d'exprimer, préalablement à l'ingestion du produit, et/ou après son ingestion, des protéines à effet prophylactique ou thérapeutique, telles que des enzymes (permettant par exemple de faciliter la digestion), des protéines permettant de stimuler le système immunitaire, des antigènes vaccinaux, etc. Dans la plupart des cas, on préférera, pour les utilisations dans ce domaine, et afin de garantir une innocuité maximale, des souches mutantes obtenues à partir de bactéries non pathogènes, et avantageusement, de bactéries habituellement utilisées pour l'alimentation. Cependant, dans le cadre d'utilisations vaccinales, on peut utiliser des souches mutantes obtenues à partir de bactéries (notamment streptocoques, staphylocoques, entérocoques ou listeria) pathogènes, et de préférence, de variants de ces bactéries portant déjà une ou plusieurs mutations atténuant leur pouvoir pathogène ; l'inactivation de la protéine HtrA, en limitant les capacités de survie de ces bactéries en conditions de stress, peut contribuer à atténuer leur virulence, comme observé précédemment dans le cas de certaines bactéries à gram-négatif.

Dans le cadre de certaines applications, dans lesquelles la bactérie hôte doit être viable et capable de produire des protéines à des températures de l'ordre de 35 à 40°C, par exemple la production en fermenteur de certaines protéines, ou la production après ingestion, dans le tractus digestif de l'homme ou d'un animal, de protéines à activité thérapeutique ou prophylactique, on utilisera avantageusement des souches mutantes obtenues à partir de bactéries thermophiles, telles que *Streptococcus thermophilus.*

La présente invention sera mieux comprise à l'aide du complément de description qui va suivre, qui se réfère à des exemples non-limitatifs, illustrant l'obtention de mutants de *L. lactis* dans lesquels la protéase de surface HtrA est inactive, et les propriétés de ces mutants.

### EXEMPLE 1 : INACTIVATION DU GENE htrA DE L. lactis

Le gène *htrA,* porté par le chromosome de la souche IL1403 (CHOPIN et al. Plasmid, 11, 260-263, 1984) de *L. lactis* subsp. *lactis,* a été inactivé par intégration d'un plasmide suicide portant un fragment interne du gène (FA) de 665 pb.

A titre de témoin positif d'intégration, on a utilisé un plasmide suicide portant un fragment tronqué en 3' (GA) de 902pb, dont l'intégration sur le chromosome restitue une copie sauvage du gène.

Ces fragments ont été préalablement obtenus par amplification PCR, à partir de l'ADN génomique de la souche IL1403 de *L. lactis* subsp. *lactis,* en utilisant les couples d'amorces F/A et G/A :
- F [5'-GGAGCCA(G/T)(A/C/T)GC(A/G/C/T)(C/T)T(A/G/T)GG-3'] localisée en aval du codon d'initiation ATG
- G [5'-GTTTCCACTTTTCTGTGG-3'] localisée en amont du promoteur de *htrA*
- A [5'-TT(A/T)CC(A/T)GG(A/G)TT(A/G/T)AT(A/G/C/T)GC-3']. localisée en amont du codon de la sérine du site catalytique.
L'emplacement des amorces, F, G, et A, est indiqué sur la figure 1.

L'amplification a été effectuée dans les conditions suivantes :
- mélange réactionnel : 0,2mM de chaque dNTP, 5µM de chaque oligonucléotide, environ 500ng d'ADN chromosomique, 2mM MgCl₂, 1,25 unité de Taq-DNA-pol (BOEHRINGER MANNHEIM) dans le tampon Taq fourni par le fabricant ;
- conditions de température : 5min 94°C, 30 cycles (30sec à 94°C, 30sec à 46°C et 30sec à 72°C), et 4°C.

Les fragments amplifiés ont été ligaturés au plasmide linéaire pGEM^{T} (PROMEGA). Après transformation de *E. coli* TG1 par les produits de ligation, les clones résistants à l'ampicilline, et dépourvus d'activité β-galactosidase sont sélectionnés. Les plasmides obtenus, portant respectivement les fragments FA et GA, sont dénommés pES1.1 et pES2.1.

Les inserts FA et GA ont été sous-clonés dans un vecteur suicide portant un gène de résistance au chloramphénicol. Ce vecteur étant incapable de se répliquer seul en l'absence de la protéine RepA qui est nécessaire à l'initiation de sa réplication, des cointégrats ont été créés par ligature entre chacun des plasmides pES1.1 et pES2.1, et le vecteur suicide, préalablement linéarisés.

Après transformation de la souche TG1 d'*E. coli*, et sélection des clones résistant au chloramphénicol, la partie pGEM^{T} des cointégrats a été délétée, et les vecteurs re-circularisés. Les plasmides obtenus sont multipliés dans la souche d'*E. coli* TG1 *repA⁺* ; après sélection des clones résistant au chloramphénicol, on obtient les plasmides suicide dénommés pVS6.1 et pVS7.4.

pVS6.1 contient le fragment FA, et pVS7.4 contient le fragment GA du gène *htrA_{Ll}* de la souche IL1403 de *L. lactis* subsp. *lactis.*

Ces plasmides ont été utilisés pour transformer la souche IL1403 de *L. lactis* subsp. *lactis* ; les clones ayant intégré ces plasmides au locus *htrA* sur le chromosome ont été sélectionnés en présence de chloramphénicol.

Dans les deux cas, plusieurs clones indépendants résistants au chloramphénicol ont été obtenus. Cinq clones de chaque classe, notés A à E dans le cas de l'intégration de pVS6.1, et 17 à 22 dans le cas de l'intégration de pVS7.4, ont été choisis pour analyse.

Pour chacun de ces clones, l'intégration au locus *htrA* a été confirmée par transfert de Southern.

Deux clones, A et 17, ont été choisis pour les analyses suivantes ; ils constituent les deux prototypes des souches mutantes, qui seront dénommées ci-après :
- *htrA* (mutation nulle du gène *htrA_{Ll},* Cm^{R}) ; cette souche n'exprime pas de protéase HtrA active ;
- *htrA⁺*/*htrA* (copie sauvage + copie tronquée du gène *htrA_{Ll},* Cm^{R}) ; cette souche exprime une protéase Htra_{Ll} active.

### EXEMPLE 2 : ROLE DU GENE htrA_{Ll} DE L. lactis DANS LA SURVIE A HAUTE TEMPERATURE

Les deux souches *htrA* et *htrA⁺*/*htrA* sont cultivées, en culture liquide, dans les conditions habituelles de croissance de *L. lactis,* c'est-à-dire à 30°C et en présence d'oxygène mais sans agitation, et en présence de chloramphénicol.
Le comportement de la souche *htrA* de *L. lactis* subsp. *lactis* à 30°C et à 37°C, a été étudié en utilisant comme témoins la souche *htrA⁺*/*htrA,* ainsi que la souche-mère IL403 (cultivée en l'absence de chloramphénicol).

Les bactéries ont été cultivées pendant 1 nuit à température ambiante, en milieu M17 contenant 1% de glucose (+2,5 µg/ml chloramphénicol pour les deux souches *htrA* et *htrA⁺*/*htrA*). Les cultures ont été diluées au 1/100^{iéme} le matin dans le même milieu, et divisées en deux lots placés en semi-anaérobiose à 30°C ou à 37°C. La croissance a été suivie par mesure de la DO₆₀₀.

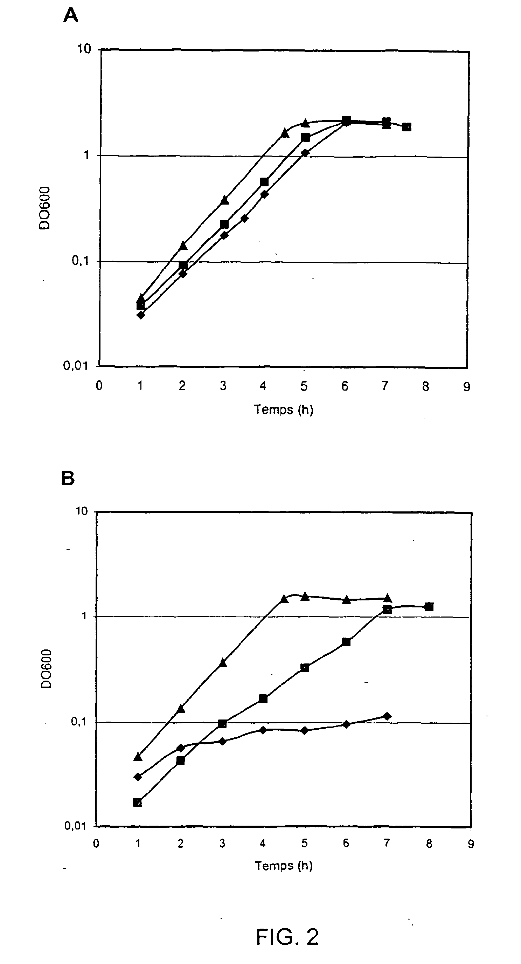

Les résultats sont illustrés par la Figure 2.

A 30°C (Fig. 2A), on constate que la souche *htrA⁺*/*htrA* (■), la souche *htrA* (◆), et la souche sauvage IL1403 (▲) présentent des temps de génération très proches : 65 min pour la souche sauvage, 70 min. pour *htrA⁺*/*htrA,* et 75 min pour *htrA* ; enfin, pour les 3 cultures, les valeurs de DO₆₀₀ correspondant à la phase stationnaire sont très comparables (DO₆₀₀ = 2.1 à 2,2).

Ces résultats indiquent qu'il n'y a pas de différence de croissance significative entre ces trois souches à 30°C.

A 37°C (Fig. 2B), la souche *htrA⁺*/*htrA* (■) a un temps de génération de 100 min, et la DO₆₀₀ de la phase stationnaire est moindre qu'à 30°C (DO₆₀₀ = 1,25). Une croissance plus faible à 37°C qu'à 30°C est également observée pour la souche sauvage IL1403 (▲) ; le temps de génération est de 65 min, mais la DO₆₀₀ de la phase stationnaire est plus faible qu'à 30°C (DO₆₀₀ = 1,9). Dans le cas de la souche *htrA* (◆) la croissance est très faible, voire nulle, et DO₆₀₀ ne dépasse pas 0,1 même après 7h de culture.

Il ressort de ces résultats que la souche *htrA* de *L. lactis* subsp. *lactis* est thermosensible, et que la mutation htrA est létale à 37°C.

### EXEMPLE 3 : ROLE DU GENE htrA_{Ll} DE L. LACTIS DANS LA PROTEOLYSE DE SURFACE

L'effet de la mutation *htrA_{Ll}* sur la stabilité de cinq protéines exportées a été testé. Ces protéines sont :
i) une protéine hétérologue, la nucléase sécrétée de *S. aureus,* Nuc ; cette protéine est exprimée par le plasmide pNuc3 (LE LOIR et al., J. Bacteriol. 176:5135-5139, 1994 ; LE LOIR et al., J. Bacteriol. 180:1895-903 1998) ;
ii) trois protéines hybrides (Usp-Δ_{SP}Nuc, Nlp4-A_{SP}Nuc, et Exp5-A_{SP}Nuc) résultant de la fusion entre le rapporteur Δ_{SP}Nuc et des fragments de protéines exportées de *L. lactis* : la protéine sécrétée Usp45 (VAN ASSELDONK et al., Gene 95:155-60, 1990), la lipoprotéine Nlp4, et la protéine Exp5 (qui est elle-même une protéine de fusion entre une protéine exportée et une protéine cytoplasmique) ; ces protéines, ainsi que les plasmides pVE8009, pVE8024 et pVE8021 qui les expriment respectivement, sont décrits par POQUET *et al*. (1998, publication précitée) ;
iii) une protéine naturellement exportée de *L. lactis,* AcmA.

Chez la souche sauvage MG1363 de *L. lactis* subsp. *cremoris,* Usp-Δ_{SP}Nuc est sécrétée, Nlp4-Δ_{SP}Nuc est associée aux cellules ; pour ces 2 protéines, on détecte dans le milieu, à côté de la forme mature, différents produits de dégradation, parmi lesquels le peptide NucA provenant de la partie Δ_{SP}Nuc de la fusion ; quant à la fusion tripartite Exp5-Δ_{SP}Nuc, elle est très instable et on ne détecte pas la forme mature dans le milieu mais seulement les produits de dégradation, dont le peptide NucA. La forme mature, ainsi que les produits de dégradation de ces trois protéines hybrides peuvent être détectées à l'aide d'anticorps anti-NucA.

La protéine naturellement exportée de *L. lactis* choisie est la bactériolysine AcmA (BUIST et al., J. Bacteriol. 177:1554-1563, 1995). Cette protéine qui dégrade le peptidoglycane est à la fois sécrétée et associée à la surface, probablement par affinité avec son substrat. Elle présente, aussi bien chez la souche MG1363 de *L. lactis* subsp. *cremoris* que chez la souche IL1403 de *L. lactis* subsp. *lactis,* des produits de protéolyse actifs et donc détectables, comme la protéine intacte, par zymogramme.

Les souches transformées par les plasmides exprimant ces différentes protéines sont cultivées à 30°C pendant plusieurs heures, au moins jusqu'au milieu de la phase exponentielle ou jusqu'au début de la phase stationnaire.

Pour chaque plasmide, des cultures des trois souches IL1403, *htrA,* et *htrA⁺*/*htrA,* ayant atteint des DO₆₀₀ comparables ont été utilisées pour extraire des échantillons protéiques : a) de la culture totale, b) des cellules, c) du milieu, selon le protocole décrit par POQUET *et al*., (1998, publication précitée).

Ces échantillons sont soumis à une électrophorèse (SDS-PAGE) sur gel dénaturant.

Pour détecter les protéines Nuc, Usp-Δ_{SP}Nuc, Nlp4-Δ_{SP}Nuc, Exp5-Δ_{SP}Nuc, et leurs produits de dégradation, on procède à un transfert des protéines sur membrane, puis à une révélation immunologique grâce à des anticorps anti-NucA, qui sont détectés à l'aide d'un conjugué protéine G/peroxydase (BIO-RAD), et d'un kit de chimioluminescence (DUPONT-NEN).

AcmA est détecté par zymogramme (BUIST *et al*., 1995, publication précitée) : des microcoques dont la paroi est sensible à AcmA sont inclus dans le gel d'électrophorèse à la concentration de 0,2%, ce qui le rend opaque ; après électrophorèse, le gel est traité à 37°C pendant une nuit dans un tampon contenant 50mM de Tris/HCl à pH7 et 0,1% de Triton X100, ce qui permet la lyse des microcoques par AcmA ou ses produits de protéolyse actifs. Le gel est ensuite coloré par du bleu de méthylène à 0,1% dans du KOH à 0,01% : les bandes correspondant à l'activité AcmA apparaissent comme des halos d'hydrolyse transparents sur fond bleu.

Pour chaque protéine, les profils de dégradation dans les souches IL1403, *htrA,* et *htrA⁺*/*htrA,* ont été comparés en observant le contenu protéique accumulé pendant plusieurs heures de culture.

Les Figures 3 à 6 présentent respectivement les résultats de détection immunologique, pour les protéines Nuc, Usp-Δ_{SP}Nuc, Nlp4-Δ_{SP}Nuc et Exp5-Δ_{SP}Nuc. Pour les protéines Nuc, (Fig.3) et Usp-Δ_{SP}Nuc, (Fig.4),

La Fig. 7 représente un zymogramme de l'activité bactériolysine d'AcmA ; la détection a été effectuée sur l'ensemble de la culture (T), les cellules seules (C) ou le milieu (M).

### Chez la souche IL1403 :

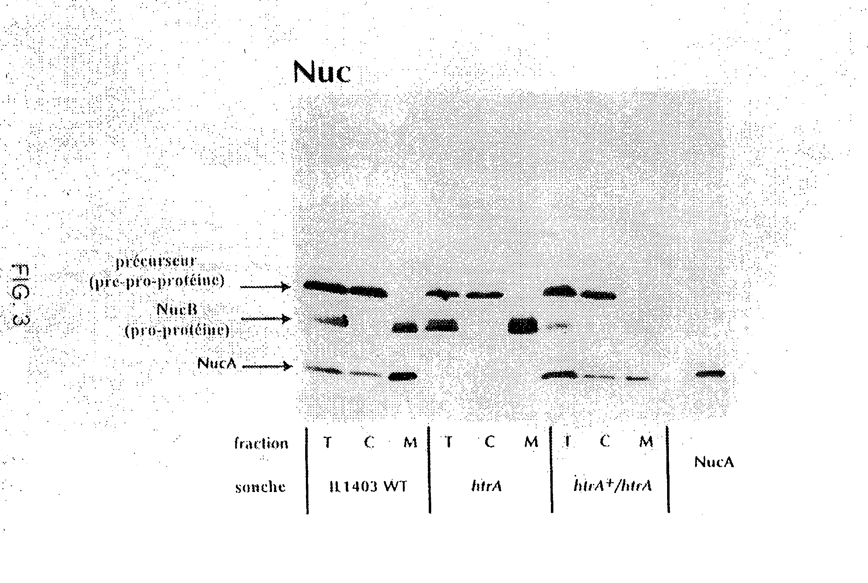

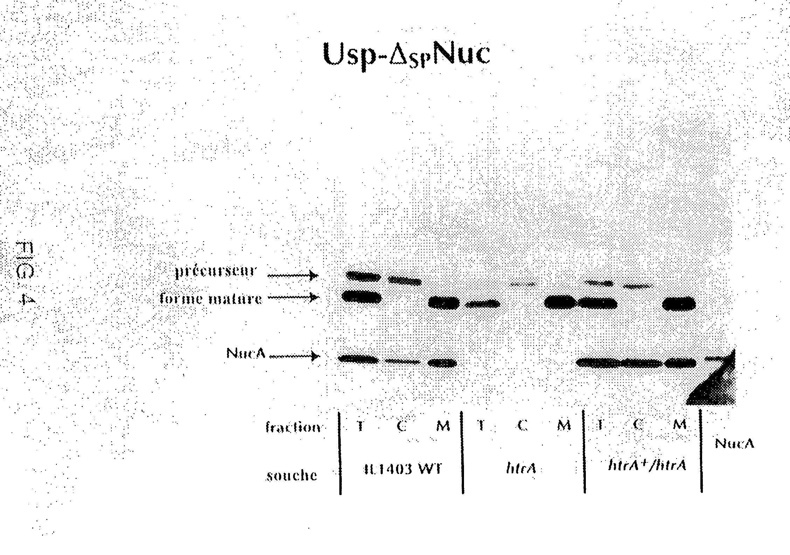

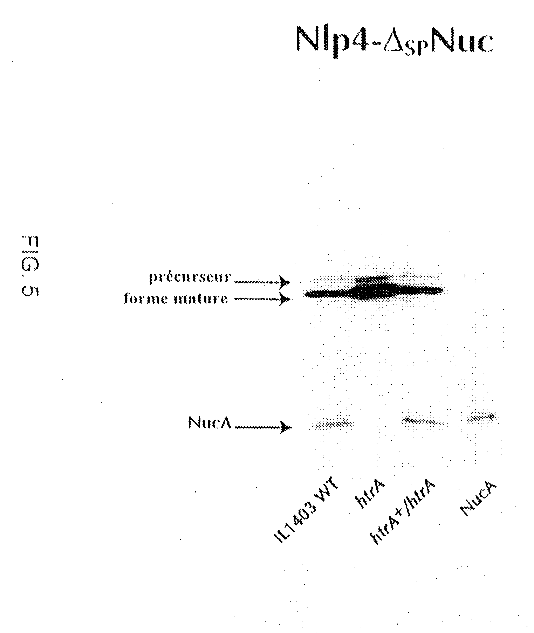

Pour les protéines sécrétées Nuc et Usp-Δ_{SP}Nuc (Fig. 3 et 4 : trois premiers puits), et pour la lipoprotéine Nlp4-Δ_{SP}Nuc (Fig.5 : premier puits), on détecte un profil de trois bandes, comme précédemment observé chez la souche MG1363 (LE LOIR *et al.,* 1994 ; POQUET *et al.,* 1998, publications précitées) :
a) celle de plus haut poids moléculaire est le précurseur dont le peptide-signal n'a pas été clivé, ce qui est confirmé par sa présence exclusive dans les cellules (Fig. 3 et 4);
b) la bande intermédiaire est la forme mature après clivage du peptide-signal, et elle est présente exclusivement dans le milieu dans le cas des protéines sécrétées Nuc et Usp-Δ_{SP}Nuc (Fig. 3 et 4) ;
c) la bande de plus faible poids moléculaire est le peptide NucA qui comigre pratiquement avec la forme commerciale NucA purifiée à partir de *S. aureus* (la légère différence de migration étant due aux spécificités de clivage distinctes chez *S. aureus* et *L. lactis*), et qui se trouve à la fois libéré dans le milieu et associé aux cellules.

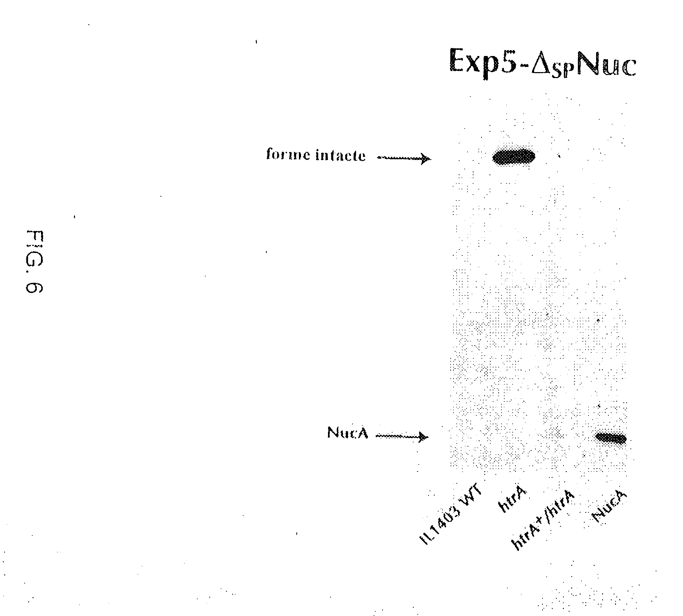

Pour la protéine Exp5-Δ_{SP}Nuc (Fig. 6 : premier puits) on ne détecte que très difficilement deux formes, une de haut poids moléculaire, et une de faible poids moléculaire, NucA, qui comigre pratiquement avec la forme purifiée commerciale ; la protéolyse chez IL1403 est donc pratiquement totale.

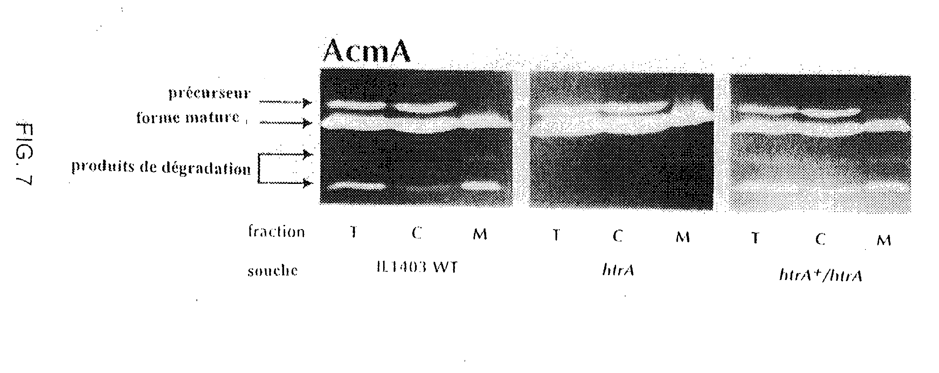

Pour la protéine AcmA (Fig. 7 : les trois premiers puits), on détecte comme précédemment observé chez la souche MG1363 (BUIST *et al*., 1995, publication précitée), un profil de quatre bandes :
a) celle de plus haut poids moléculaire est le précurseur dont le peptide-signal n'a pas été clivé, qui est présent exclusivement dans les cellules;
b) la bande de poids moléculaire légèrement inférieur est la forme mature après clivage du peptide-signal, qui est à la fois sécrétée dans le milieu et associée à la surface des cellules par affinité pour son substrat;
c et d) les deux bandes de plus faible poids moléculaire sont des produits de protéolyse actifs, à la fois sécrétés dans le milieu et associés à la-surface des cellules par affinité pour leur substrat.

### Chez la souche htrA⁺/htrA :

(Fig. 3 et 4 : trois derniers puits, Fig.5 et 6 : dernier puits, et Fig. 7 : trois derniers puits). Les profils observés sont absolument identiques à ceux observés dans la souche sauvage. La souche *htrA⁺*/*htrA* présente donc un phénotype de protéolyse sauvage, s'expliquant par la copie sauvage du gène *htrA_{Ll}* qu'elle possède.

### Chez la souche htrA :

(Fig. 3 et 4 : trois puits centraux, Fig.5 et 6 : puits central, et Fig. 7 : trois puits centraux).

Dans tous les cas, on ne détecte aucun des produits de protéolyse ; simultanément, la quantité de protéine mature (ou de haut poids moléculaire dans le cas de Exp5-Δ_{SP}Nuc) augmente.

Ces résultats montrent que le produit du gène *htrA_{Ll}* est bien responsable de la dégradation des protéines sécrétées, et que son inactivation entraîne l'abolition totale de cette dégradation.

## Revendications

1. Procédé pour la production d'une protéine d'intérêt, comprenant la mise en culture d'une bactérie qui exprime ladite protéine d'intérêt et l'obtention de ladite protéine d'intérêt exportée par ladite bactérie, **caractérisé en ce que** ladite bactérie est une bactérie à gram positif choisie parmi les *Streptococcaceae,* les *Lactobacillaceae,* les *Bacillaceae* des genres *Staphylococcus* et *Listeria,* et les *Enterococcaceae* du genre *Enterococcus,* dont la protéase de surface HtrA, est inactivée par mutation.

2. Procédé selon la revendication 1, **caractérisée en ce que** ladite bactérie à gram positif est choisie dans le groupe constitué par *Lactococcus* spp., *Lactobacillus* spp., et *Streptococcus thermophilus.*

3. Procédé selon une quelconque des revendications 1 ou 2, **caractérisé en ce que** ladite bactérie à gram positif est choisie dans le groupe constitué par *Lactococcus* spp., et est dépourvue d'activité protéasique PrtP.

4. Bactérie à gram positif telle que définie dans lés revendications 1 à 3, **caractérisée en ce qu'**elle comprend au moins une cassette d'expression d'un gène d'intérêt, à l'exception d'une souche de *Lactobacillus helveticus* comprenant une seule cassette d'expression constituée par la séquence codant pour lé gène rapporteur *gusA* insérée dans le gène *htrA* de ladite souche de *Lactobacillus helveticus* sous contrôle transcriptionnel du promoteur dudit gène *htrA.*

5. Utilisation d'une bactérie telle que définie dans une quelconque des revendications 2. à 3, pour la préparation d'un produit fermenté.

6. Utilisation d'une bactérie telle que définie une quelconque des revendications 2 à 3, pour la préparation d'un aliment diététique.

7. Utilisation d'une bactérie telle que définie une quelconque des revendications 1 à 3, pour la préparation d'un médicament.

8. Utilisation selon la revendication 7, **caractérisée en ce que** ledit médicament est un vaccin.

## Claims

1. Process for the production of a protein of interest, comprising the culturing of a bacterium which expresses the said protein of interest and the obtaining of the said protein of interest exported by the said bacterium, **characterised in that** the said bacterium is a gram-positive bacterium selected from the *Streptococcaceae,* the *Lactobacillaceae,* the *Bacillaceae* of the genera Staphylococcus and Listeria, and the *Enterococcaceae* of the genus Enterococcus, whose HtrA surface protease is inactivated by mutation.

2. Process according to claim 1, **characterised in that** the said gram-positive bacterium is selected from the group comprising *Lactococcus* spp., *Lactobacillus* spp., and *Streptococcus thermophilus.*

3. Process according to either claim 1 or claim 2, **characterised in that** the said gram-positive bacterium is selected from the group comprising *Lactococcus* spp., and is free from PrtP protease activity.

4. Gram-positive bacterium as defined in claims 1 to 3, **characterised in that** it comprises at least one expression cassette for the expression of a gene of interest, with the exception of a strain of *Lactobacillus helveticus* comprising a single expression cassette composed of the sequence encoding the *gusA* reporter gene inserted in the *htrA* gene of the said strain of *Lactobacillus helveticus* under the transcriptional control of the promoter of the said *htrA* gene.

5. Use of a bacterium as defined in either claim 2 or claim 3, for the preparation of a fermented product.

6. Use of a bacterium as defined in either claim 2 or claim 3, for the preparation of a dietary foodstuff.

7. Use of a bacterium as defined in any one of claims 1 to 3, for the preparation of a medicament.

8. Use according to claim 7, **characterised in that** the said medicament is a vaccine.

## Patentansprüche

1. Verfahren zur Herstellung eines gewünschten Proteins, umfassend die Kultivierung eines Bakteriums, dass das oben genannte gewünschten Protein ausdrückt, und die Gewinnung des durch das oben genannte Bakterium exportierte gewünschte Protein, **dadurch gekennzeichnet, dass** das oben genannte Bakterium ein grampositives Bakterium ist, das zu den Gruppen *Streptococcaceae, Lactobacillaceae* und *Bacillaceae* der Gattungen *Staphylococcus* und *Listeria* bzw. zu den *Enterococcaceae* der Gattung *Enterococcus* gehört, deren Oberflächenprotease HtrA durch Mutation inaktiviert wird.

2. Verfahren gemäß Anspruch 1, **dadurch gekennzeichnet, dass** das oben genannte grampositive Bakterium aus der Gruppe gewählt wird, die durch *Lactococcus* spp., *Lactobacillus* spp. und *Streptococcus thermophilus* gebildet ist.

3. Verfahren gemäß einem der Ansprüche 1 oder 2, **dadurch gekennzeichnet, dass** das oben genannte grampositive Bakterium aus der Gruppe gewählt wird, die durch *Lactococcus* spp. gebildet wird und keine proteasische Aktivität hinsichtlich *PrtP* aufweist.

4. Grampositives Bakterium wie in den Ansprüchen 1 bis 3 definiert, **dadurch gekennzeichnet, dass** es mindestens eine Expressionskassette für ein gewünschtes Gen umfasst, mit Ausnahme eines Stamms von *Lactobacillus helveticus,* der eine einzige Expressionskassette umfasst, die durch die Codierfolge für das Reportergen *gusA* gebildet ist, welche unter transkriptionaler Kontrolle des Promoters des oben genannten htrA-Gens in das *htrA*-Gen des oben genannten Stamms von *Lactobacillus helveticus* eingegeben wird.

5. Verwendung eines in einem der Ansprüche 2 bis 3 definierten Bakteriums für die Zubereitung eines Gärungsprodukts.

6. Verwendung eines in einem der Ansprüche 2 bis 3 definierten Bakteriums für die Zubereitung eines dietätischen Lebensmittels.

7. Verwendung eines in einem der Ansprüche 1 bis 3 definierten Bakteriums für die Zubereitung eines Medikaments.

8. Verwendung gemäß Anspruch 7, **dadurch gekennzeichnet, dass** das Medikament ein Impfstoff ist.


### LISTE DE SEQUENCES

<110> INSTITUT NATIONAL DE LA RECHERCHE AGRONOMIQUE (INRA)
   POQUET, Isabelle
   GRUSS, Alexandra
   BOLOTINE, Alexandre
   SOROKINE, Alexei
<120> BACTERIES A GRAM POSITIF DEPOURVUES D'ACTIVITE
   PROTEASIQUE HtrA, ET LEURS UTILISATIONS.
<130> MJPcb539/89
<140>
   <141>
<150> FR9816462
   <151> 1998-12-24
<160> 2
<170> PatentIn Ver. 2.1
<210> 1
   <211> 1740
   <212> ADN
   <213> Lactococcus lactis
<220>
   <221> CDS
   <222> (230)..(1453)
<400> 1
   aaacaagatg aaaacatgat ttatcaacat ttttttactt ttttccactt ttctgtggaa 60
   aactttatta aaatatccac ttatcctcat taatttttag attatccaca aaaatgtgga 120
   gaaactatat tagtttgatt tttgttacta ttaaggtatt attaagtgag agtagatata 180
   attacatcat agaaatgcta caaagattaa taattgaaag gaattattt atg gca aaa 238
                                                         Met Ala Lys
                                                          1
   gct aat ata gga aaa ttg cta tta aca ggt gtc gtg ggc gga gcc atc   286
   Ala Asn Ile Gly Lys Leu Leu Leu Thr Gly Val Val Gly Gly Ala Ile
        5                  10                  15
   gca ctt gga gga agt gca atc tat caa agc act aca aat caa tcg gca   334
   Ala Leu Gly Gly Ser Ala Ile Tyr Gln Ser Thr Thr Asn Gln Ser Ala
    20                  25                  30                  35
   aat aat agt cgt tca aat aca act agt aca aag gtt agt aac gtt tcg   382
   Asn Asn Ser Arg Ser Asn Thr Thr Ser Thr Lys Val Ser Asn Val Ser
                    40                  45                  50
   gta aat gtc aat acc gat gtt acc tct gca att gaa aaa gtt tca aat   430
   Val Asn Val Asn Thr Asp Val Thr Ser Ala Ile Glu Lys Val Ser Asn
                55                  60                  65
   tct gtc gtt tct gtt atg aat tat caa aaa gat aac tca caa agt agt   478
   Ser Val Val Ser Val Met Asn Tyr Gln Lys Asp Asn Ser Gln Ser Ser
            70                  75                  80
   gac ttc agt tca att ttt ggt gga aat agc ggt tca agt tca tcg act    526
   Asp Phe Ser Ser Ile Phe Gly Gly Asn Ser Gly Ser Ser Ser Ser Thr
        85                  90                  95
   gat ggc tta cag ctt tct agt gaa ggc tct ggt gtc atc tac aaa aaa    574
   Asp Gly Leu Gln Leu Ser Ser Glu Gly Ser Gly Val Ile Tyr Lys Lys
   100                 105                 110                 115
   tct ggt ggt gat gcc tac gtt gta act aac tac cac gtt att gct ggt    622
   Ser Gly Gly Asp Ala Tyr Val Val Thr Asn Tyr His Val Ile Ala Gly
                   120                 125                 130
   aat agc tca ctt gat gtt ctg ctt tct ggt gga caa aaa gtc aaa gat    670
   Asn Ser Ser Leu Asp Val Leu Leu Ser Gly Gly Gln Lys Val Lys Asp
               135                 140                 145
   tct gtg gtt ggt tat gat gaa tac aca gac ctt gct gtt ctt aaa atc    718
   Ser Val Val Gly Tyr Asp Glu Tyr Thr Asp Leu Ala Val Leu Lys Ile
           150                 155                 160
   agt tct gaa cat gtc aaa gat gtg gcg aca ttc gct gat tct agt aaa    766
   Ser Ser Glu His Val Lys Asp Val Ala Thr Phe Ala Asp Ser Ser Lys
       165                 170                 175
   tta aca att ggt gaa cct gcc att gcc gtt ggc tca cct tta ggt agt    814
   Leu Thr Ile Gly Glu Pro Ala Ile Ala Val Gly Ser Pro Leu Gly Ser
   180                 185                 190                 195
   caa ttt gca aac acc gca act gaa gga att tta tct gca aca agc cgt    862
   Gln Phe Ala Asn Thr Ala Thr Glu Gly Ile Leu Ser Ala Thr Ser Arg
                   200                 205                 210
   caa gtg act ttg acc caa gaa aat ggt caa aca act aat atc aat gca    910
   Gln Val Thr Leu Thr Gln Glu Asn Gly Gln Thr Thr Asn Ile Asn Ala
               215                 220                 225
   att caa aca gat gct gcc att aac cct ggt aac tct gga ggg gct ttg    958
   Ile Gln Thr Asp Ala Ala Ile Asn Pro Gly Asn Ser Gly Gly Ala Leu
           230                 235                 240
   att aat att gaa gga caa gtt att gga att act caa agt aaa att aca    1006
   Ile Asn Ile Glu Gly Gln Val Ile Gly Ile Thr Gln Ser Lys Ile Thr
       245                 250                 255
   aca act gaa gat ggt tct act tct gtc gaa ggt tta gga ttt gcg att    1054
   Thr Thr Glu Asp Gly Ser Thr Ser Val Glu Gly Leu Gly Phe Ala Ile
   260                 265                 270                 275
   cct tct aat gat gtc gta aat atc att aat aaa ctt gaa gat gat ggt    1102
   Pro Ser Asn Asp Val Val Asn Ile Ile Asn Lys Leu Glu Asp Asp Gly
                   280                 285                 290
   aag att tca cgc cct gct tta ggt atc cga atg gtt gac ctt tca caa    1150
   Lys Ile Ser Arg Pro Ala Leu Gly Ile Arg Met Val Asp Leu Ser Gln
               295                 300                 305
   tta tca aca aat gac agt tct caa ttg aaa tta cta agc agt gta aca    1198
   Leu Ser Thr Asn Asp Ser Ser Gln Leu Lys Leu Leu Ser Ser Val Thr
           310                 315                 320
   ggt ggg gtt gtt gtt tac tcc gtc caa tct gga ctt cct gct gcc tca    1246
   Gly Gly Val Val Val Tyr Ser Val Gln Ser Gly Leu Pro Ala Ala Ser
       325                 330                 335
   gct ggt ttg aaa gct gga gat gta att aca aag gtt ggc gat aca gca    1294
   Ala Gly Leu Lys Ala Gly Asp Val Ile Thr Lys Val Gly Asp Thr Ala
   340                 345                 350                 355
   gta acc tct tca aca gac ttg caa agt gct ctt tac tca cac aat atc    1342
   Val Thr Ser Ser Thr Asp Leu Gln Ser Ala Leu Tyr Ser His Asn Ile
                   360                 365                 370
   aat gat aca gta aaa gtt act tat tat cgt gat ggt aaa tca aat aca    1390
   Asn Asp Thr Val Lys Val Thr Tyr Tyr Arg Asp Gly Lys Ser Asn Thr
               375                 380                 385
   gca gat gtt aaa ctt tct aaa tca acc agt gac tta gaa aca agc agt    1438
   Ala Asp Val Lys Leu Ser Lys Ser Thr Ser Asp Leu Glu Thr Ser Ser
           390                 395                 400
   cca tct tct tct aat taataactta ataatttaat aaaagtcttc tgtaaataga    1493
   Pro Ser Ser Ser Asn
       405
   aggctttttt catactaaag tctgaaattt ttaaaaataa taaatttcca tttttctttt 1553
   attgatttat ggtaaaataa agttaagcat gaaaatttta ctttacttag aagccgaaca 1613
   atttttgagt cattcaggaa ttggtcgtgc aatgaaacat caacaacgcg cccttgattt 1673
   aatgggcatt gactggacaa aaaatcctga ggatgattac gatatcctcc atttaaatac 1733
   ttatggc                                                           1740
<210> 2
   <211> 408
   <212> PRT
   <213> Lactococcus lactis
<400> 2